(19) Europäisches Patentamt
European Patent Office
Office européen des brevets

(11) Veröffentlichungsnummer: **0 308 839**
**A2**

# (12) EUROPÄISCHE PATENTANMELDUNG

(21) Anmeldenummer: 88115289.6

(22) Anmeldetag: 17.09.88

(51) Int. Cl.4: C07C 103/737 , C07C 131/02 , C07C 61/13 , C07C 87/40 , C07D 295/18 , C07D 295/06 , C07C 121/46 , C07C 69/753 , A61K 31/16 , A61K 31/135 , A61K 31/19

Patentansprüche für folgende Vertragsstaaten: ES + GR.

(30) Priorität: 23.09.87 DE 3731913

(43) Veröffentlichungstag der Anmeldung: 29.03.89 Patentblatt 89/13

(84) Benannte Vertragsstaaten: AT BE CH DE ES FR GB GR IT LI LU NL SE

(71) Anmelder: HOECHST AKTIENGESELLSCHAFT
Postfach 80 03 20
D-6230 Frankfurt am Main 80(DE)

(72) Erfinder: Siegel, Herbert, Dr.
Am Alten Birnbaum 10a
D-6238 Hofheim am Taunus(DE)
Erfinder: Granzer, Ernold, Dr. Dr.
Falkensteiner Strasse 24
D-6233 Kelkheim (Taunus)(DE)

## (54) Pharmazeutische Präparate enthaltend ein 7-Diphenylmethylenbicycloheptan-oder 7-Diphenylmethylenbicyclohepten-Derivat, die Verwendung dieser Verbindungen als Arzneimittel sowie neue 7-Diphenylmethylenbicycloheptane und 7-Diphenylmethylenbicycloheptene und Verfahren zu ihrer Herstellung.

(57) Pharmazeutische Präparate enthaltend ein 7-Diphenylmethylenbicycloheptan- oder 7-Diphenylmethylenbicyclohepten-Derivat der Formel I

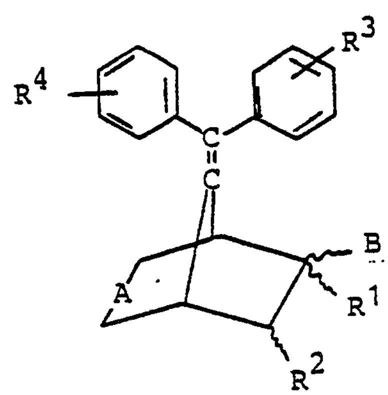

I

worin A, B, $R^1$, $R^2$, $R^3$ und $R^4$ die angegebenen Bedeutungen haben, oder ein physiologisch verträgliches Säureadditionssalz dieser Verbindungen sowie deren Verwendung als Hypolipidämikum werden beschrieben. Außerdem werden neue 7-Diphenylmethylenbicycloheptan- und -hepten-Derivate der Formel Ia

EP 0 308 839 A2

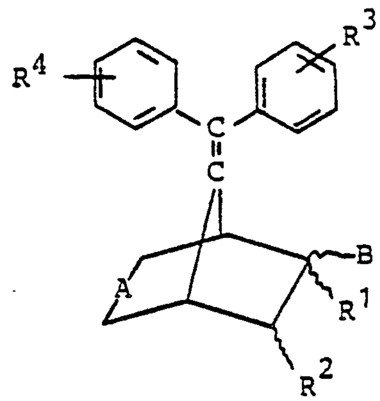

Ia

worin A, B, $R^1$, $R^2$, $R^3$, $R^4$ die angegebenen Bedeutungen haben, und deren physiologisch verträgliche Säureadditionssalze sowie Verfahren zu ihrer Herstellung beschrieben.

# Pharmazeutische Präparate enthaltend ein 7-Diphenylmethylenbicycloheptan- oder 7-Diphenylmethylenbicyclohepten-Derivat, die Verwendung dieser Verbindungen als Arzneimittel sowie neue 7-Diphenylmethylenbicycloheptane und 7-Diphenylmethylenbicycloheptene und Verfahren zu ihrer Herstellung

Die Erfindung betrifft pharmazeutische Präparate, die 7-Diphenylmethylenbicycloheptane bzw. -ene enthalten sowie die Verwendung dieser Verbindungen als Arzneimittel, insbesondere zur Behandlung von Hyperlipidämien. Die Erfindung betrifft ferner neue 7-Diphenylmethylenbicycloheptane und 7-Diphenylmethylenbicycloheptene und ein Verfahren zu ihrer Herstellung.

Es wurde bereits beschrieben, daß 7-Diphenylmethylenbicycloheptane und -heptene, die in 2-Stellung direkt oder über eine Kette von Kohlenstoffatomen mit einem Imidazolrest am Stickstoff verbunden sind, eine hypolypidämische Wirkung aufweisen. Diese Verbindungen sind Gegenstand der deutschen Patentanmeldung DE-A 34 10 498.4. (entspr. EP-A-O 158 144 bzw. US-Patentschrift 4 647 575).

Es wurde nun überraschenderweise gefunden, daß 7-Diphenylmethylenbicycloheptane- und -hepten-Derivate, die nicht Imidazol-substituiert sind, ebenfalls eine sehr starke hypolipidämische Wirkung aufweisen. Bei den aktiven Verbindungen handelt es sich um7-Diphenylmethylenbicyclo-heptane bzw. -heptene, welche in 2-Stellung mit Sauerstoff-oder Stickstoff-haltigen funktionellen Gruppen substituiert sind.

Die Erfindung betrifft daher pharmazeutische Präparate enthaltend ein 7-Diphenylmethylenbicycloheptan bzw. -hepten der allgemeinen Formel I

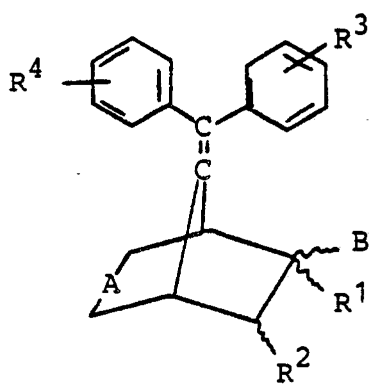

I

worin
A eine Einfach- oder Doppelbindung ist,
B eine Carbonsäureamidgruppe der Formel

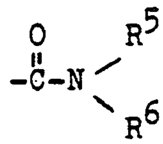

oder eine Methylenaminogruppe der Formel

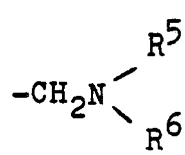

ist,
worin $R^5$ und $R^6$ gleich oder verschieden sind und Wasserstoff, oder $C_1$-$C_4$-Alkyl bedeuten oder worin $R^5$

und $R^6$ zusammen mit dem Stickstoffatom einen Morpholin-Piperazin- oder einen in 4-Stellung mit $C_1$-$C_4$-Alkyl substituierten Piperazinring bilden, oder B ist -OH, -CN, $-CO_2H$, $-CO_2-(C_1-C_4)$-Alkyl oder $-\underset{\underset{O}{\|}}{C}-(C_1-C_4)$-Alkyl,

$R^1$ Wasserstoff, Chlor, $C_1$-$C_4$-Alkyl oder zusammen mit B eine Oximgruppe (= N-OH) oder über eine Doppelbindung gebundener Sauerstoff (= O) ist,
$R^2$ Wasserstoff oder zusammen mit B ein Dicarbonsäureanhydrid
$(-\overset{\overset{O}{\|}}{C}-O-\overset{\overset{O}{\|}}{C}-)$ oder ein Dicarbonsäureimid
$(-\overset{\overset{O}{\|}}{C}-NH-\overset{\overset{O}{\|}}{C}-)$ ist,
$R^3$ und $R^4$ gleich oder verschieden sind und Wasserstoff, Halogen, $(C_1$-$C_4)$-Alkyl, $(C_1$-$C_4)$-Alkoxy, Trifluormethyl, Hydroxy, Amino, $(C_1$-$C_4)$-Alkylamino oder $(C_1$-$C_4)$-Dialkylamino bedeuten,
oder im Fall von basischen Substituenten für B deren physiologisch verträgliche Säureadditionssalze.

Die Erfindung betrifft ferner dieVerwendung der Verbindung der Formel I als Arzneimittel, insbesondere als Hypolipidämika, sowie die Verwendung zur Herstellung von Arzneimitteln.

Die Schlangenlinien in Formel I sowie in den folgenden Formeln besagen, daß die Substituenten sowohl in der endo-als auch in der exo-Stellung am Bicyclus stehen können.

Die Alkyl- und Alkoxyreste sind geradkettig oder verzweigt.

Bevorzugt sind als Wirkstoffe Verbindungen der Formel I, worin
A eine Einfachbindung
B eine $-CO_2H$, -CN, $-CH_2OH$ oder -OH-Gruppe
$R^1$ Wasserstoff oder zusammen mit B über eine Doppelbindung gebundener Sauerstoff (= 0) oder die Oximgruppe (= N-OH)
$R^2$ Wasserstoff
$R^3$ und $R^4$ Wasserstoff oder Halogen
bedeuten.

Die Verbindungen der Formel I sind zum Teil neu. Die Erfindung betrifft daher auch neue 7-Diphenylmethylenbicycloheptane und 7-Diphenylmethylenbicycloheptane der Formel Ia

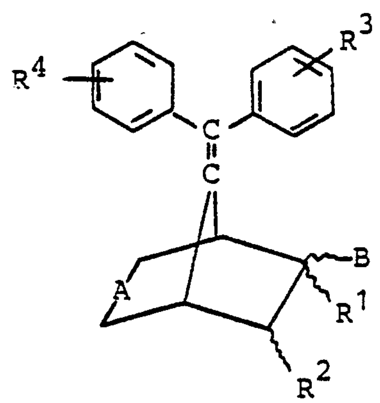

Ia

worin
A eine Einfach- oder Doppelbindung ist,
B eine Carbonsäureamidgruppe der Formel

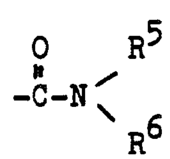

oder eine Methylenaminogruppe der Formel

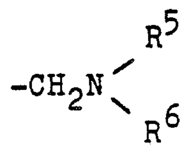

ist,
worin $R^5$ und $R^6$ gleich oder verschieden sind und Wasserstoff oder $C_1$-$C_4$-Alkyl bedeuten, oder worin $R^5$ und $R^6$ zusammen mit dem Stickstoffatom einen Morpholin-Piperazin- oder einen in 4-Stellung mit $C_1$-$C_4$-Alkyl substituierten Piperazinring bilden,
$R^1$ Wasserstoff, Chlor oder zusammen mit B eine Oximgruppe (= N-OH) ist,
$R^2$ Wasserstoff darstellt und
$R^3$ und $R^4$ gleich oder verschieden sind und Wasserstoff, Halogen, ($C_1$-$C_4$)-Alkyl, ($C_1$-$C_4$)-Alkoxy, Trifluormethyl, Hydroxy, Amino, ($C_1$-$C_4$)-Alkylamino oder ($C_1$-$C_4$)-Dialkylamino bedeuten,
sowie deren physiologisch verträgliche Säureadditionssalze.

Verbindungen der Formel Ia, worin A eine Einfachbindung ist, B und $R^1$ gemeinsam die Oximgruppe bilden und $R^3$ und $R^4$ Wasserstoff oder Chlor bedeuten, sind bevorzugt.

Die Erfindung betrifft auch Verfahren zur Herstellung von Verbindungen der Formel Ia, die dadurch gekennzeichnet sind, daß man

A) eine Bicycloheptan- oder Bicycloheptensäure der Formel Ib

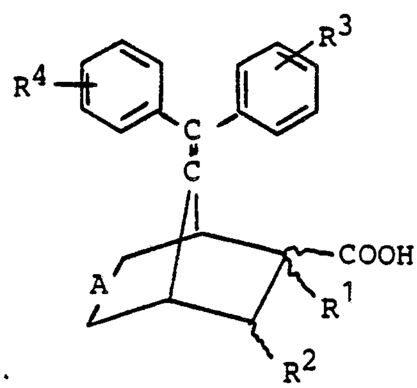

Ib

mit einem Chlorierungsmittel in das entsprechende Säurechlorid überführt und dann mit einem Amin

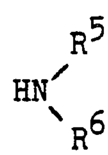

umsetzt zu einer Verbindung der Formel Ia worin A, $R^2$, $R^3$ und $R^4$ die angegebenen Bedeutungen haben, $R^1$ Wasserstoff oder Chlor ist und B die

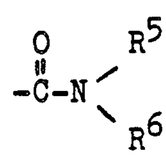

Gruppe darstellt und gegebenenfalls ein erhaltenes Carbonsäureamidderivat reduziert zu einer Verbindung der Formel Ia, worin A, $R^2$, $R^3$ und $R^4$ die angegebenen Bedeutungen haben, $R^1$ Wasserstoff oder Chlor ist

und B die

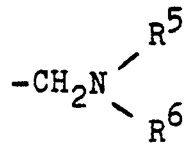

Gruppe darstellt, oder

B) ein Keton der Formel Ic

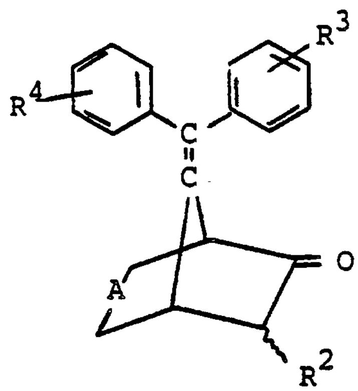

Ic .

mit Hydroxylaminhydrochlorid umsetzt zu einer Verbindung der Formel Ia, worin A, $R^2$, $R^3$ und $R^4$ die angegebenen Bedeutungen haben und $R^1$ und B gemeinsam die =NOH-Gruppe bilden,
und gegebenenfalls in einer erhaltenen Verbindung der Formel Ia, worin A eine Doppelbindung darstellt, die Doppelbindung hydriert, und gegebenenfalls eine Verbindung der Formel Ia in ein physiologisch verträgliches Säureadditionssalz überführt.

Die Verbindungen der Formel Ib sind entweder in der Literatur beschrieben (vgl. z.B. Synthesis 1985, 798 und Ann. 566, S. 27 ff (1950)) oder werden analog den dort beschriebenen Methoden hergestellt.

Als Chlorierungsmittel sind z.B. Thionylchlorid oder Phosgen geeignet.

Falls A eine Doppelbindung darstellt, so kann diese nach an sich bekannten Methoden, z.B. mit 5 % Platin auf Kohle, hydriert werden. Bei der Herstellung von Verbindungen der Formel Ia nach Verfahren A ist es zweckmäßig, die Bicycloheptencarbonsäure zur Bicycloheptancarbonsäure zu hydrieren. Die entstandene Bicycloheptancarbonsäure wird anschließend in an sich bekannter Weise z.B. in einer Eintopfreaktion mit Thionylchlorid in das entsprechende Säurechlorid überführt und dieses anschließend mit Amin zum Carbonsäureamid umgesetzt.

Die Reduktion der nach Verfahren A erhaltenen Carbonsäureamide führt man zweckmäßigerweise mit Lithiumaluminiumhydrid in einem aprotischen Lösungsmittel wie Tetrahydrofuran durch.

Die Ketone der Formel Ic, die gemäß Verfahren B als Ausgangsverbindungen eingesetzt werden, sind entweder in der Literatur beschrieben (vgl. z.B. Synthesis 1985, 798 und EP-A-O 158 144) oder können analog den beschriebenen Methoden hergestellt werden.

Die Umsetzung mit Hydroxylaminhydrochlorid erfolgt vorzugsweise in Gegenwart von Kaliumcarbonat in Ethanol als Lösungsmittel.

Aus den Aminen der allgemeinen Formel Ia können Säureadditionssalze hergestellt werden. Dazu kommen alle Säuren, die physiologisch verträgliche Salze bilden infrage. Dazu gehören sowohl anorganische Säuren wie z.B. Chlorwasserstoffsäuren, Salpetersäure und Schwefelsäure als auch mono- und bifunktionelle organische Säuren, insbesondere Carbonsäuren wie Essigsäure, Bernsteinsäure, Weinsäure usw.

Verbindungen der Formel I sind, sofern sie nicht unter Formel Ia, fallen, in der Literatur beschrieben (vgl. z.B. EP-A O 158 144, Synthesis 1985, 798, Ann. 566 27 ff (1950) und J. Med. Pharm. Chem. 5, 883 (1962)) oder können analog den beschriebenen Methoden hergestellt werden.

Die Verbindungen der Formel I besitzen wertvolle pharmakologische Eigenschaften, insbesondere zeigen sie eine sehr starke und günstige Beeinflussung der Serumlipoproteine. Die Erfindung betrifft daher pharmazeutische Präparate auf Basis dieser Verbindungen und ihre Verwendung, insbesondere zur Beeinflussung der Serumlipoproteine.

Es ist allgemein anerkannt, daß für die Entstehung arteriosklerotischer Gefäßveränderungen, insbesondere der coronaren Herzkrankheit, Hyperlipoproteinämien einen wesentlichen Risikofaktor darstellen. Für die Prophylaxe und die Regression von arteriosklerotischen Veränderungen kommt daher der Senkung erhöhter Serum-Lipoproteine eine außerordentliche Bedeutung zu. Hierbei kommt es aber auf ganz bestimmte Klassen von Serum-Lipoproteinen an, da die low density (LDL) und very low density-Lopoproteine (VLDL) einen atherogenen Risikofaktor darstellen, während die high density-Lipoproteine (HDL) eine Schutzfunktion gegenüber arteriosklerotischer Gefäßveränderung ausüben. Hypolipidämika sollen demnach VLDL-Cholesterin und LDL-Cholesterin im Serum erniedrigen, dabei aber die HDL-Cholesterin-Konzentration nach Möglichkeit unbeeinflußt lassen, sodaß der antiatherogene

$$\text{Index}\ \frac{\text{HDL-C}}{\text{LDL-C}}$$

im Vergleich zu normalen Kontrollen nicht unter 1 absinkt.

Die Verbindungen der Formel I haben wertvolle therapeutische Eigenschaften. So erniedrigen sie vor allem die Konzentration von LDL und VLDL, die HDL-Fraktion aber nur in überhöhter Dosierung, die bereits die LDL-Cholesterinkonzentrationen um mehr als ca. 50 % vermindern, sodaß im therapeutisch nutzbaren Bereich eine starke Verminderung der LDL-Fraktion ohne Beeinflussung der HDL-Fraktion resultiert. Diese Verbindungen stellen daher einen wesentlichen Fortschritt gegenüber der Vergleichsverbindung Clofibrat dar, das neben LDL immer eine sehr starke Verminderung der HDL bewirkt, wie aus den nachfolgend beschriebenen Daten ersichtlich ist. Die Verbindungen der Formel I können daher zur Prophylaxe und Regression von arteriosklerotischen Veränderungen herangezogen werden, indem sie einen kausalen Risikofaktor ausschalten. Hierzu zählen nicht nur die primären Hyperlipidämien, sondern auch gewisse sekundäre Hyperlipidämien wie sie z.B. beim Diabetes vorkommen. Die Wirkung der angeführten Verbindungen auf die Serum-Lipoproteine wurde an männlichen Wistar-Ratten untersucht, die 7 Tage per Schlundsonde mit den angeführten in Polyäthylenglykol 400 suspendierten Verbindungen behandelt wurden. Außerdem wurde eine Kontrollgruppe, die nur das Lösungsmittel Polyäthylenglykol 400 erhielt, mitgeführt, sowie bei den meisten Versuchen eine Ratten-Gruppe mit dem Standard-Hypolipidämikum Clofibrat in der Dosis von 100 mg/ kg/Tag; an der männlichen Ratte ist dies die minimale Wirkdosis. Pro Gruppe wurden in der Regel 10 Tiere eingesetzt, denen am Ende der Behandlung das Blut nach leichter Äthernarkose aus dem Orbitalplexus entnommen wurde. Aus dem gowonnenen Ratten-Serum wurden die Serum-Lipoproteine in der präparativen Ultrazentrifuge in folgende Dichteklassen getrennt:
VLDL < 1.006; LDL 1,006 bis 1,04; HDL 1,04 bis 1,21

Da im Gegensatz zum Menschen die Serumlipoproteine der Ratte etwa 4/5 HDL-Cholesterin und nur 1/5 LDL-Cholesterin enthalten und nur sehr geringe Mengen von VLDL (beim Menschen umgekehrt etwa 4/5 LDL und VLDL und nur 1/5 HDL), setzt die Beurteilung einer hypolipidämischen Wirkung an der Ratte die Fraktionierung des Rattenserums in Lipoproteinklassen voraus. Eine bloße Erniedrigung des Serum-Totalcholesteringehaltes an der Ratte würde nämlich nur die unerwünschte Erniedrigung der bei der Ratte vorwiegenden antiatherogenen HDL-Klasse anzeigen. Eine erwünschte Erniedrigung von LDL bei gleichzeitiger erwünschter Vermehrung von HDL hätte aber keine (wesentliche) Auswirkung auf den Totalcholesteringehalt des Ratten-Serums.

Aus den in der Ultrazentrifuge isolierten Lipoproteinfraktionen wurde das darin enthaltene Cholesterin vollenzymatisch nach der CHOD-PAP-Methode mittels der Testkombination von Boehringer-Mannheim bestimmt und die Werte in µg/ml Serum umgerechnet. In der angeführten Tabelle I ist die prozentuale Veränderung des Lipoprotein-Cholesterins in der behandelten Gruppe gegenüber einer unter gleichen Bedingungen mitgeführten Kontrollgruppe angegeben. Wie aus der Tabelle I ersichtlich, bewirkt Clofibrat eine ähnlich starke Verminderung der HDL- wie der LDL-Fraktion, sodaß der antiatherogene Index bei 1 bleibt, während die Verbindungen der Formel I eine starke selektiv senkende Wirkung auf die atherogenen Lipoproteinfraktionen (VLDL und LDL) ausüben und die schützende HDL-Fraktion (soweit nicht überdosiert) im wesentlichen unbeeinflußt lassen. Die untersuchten Verbindungen haben außerdem eine wesentlich höhere Efficacy als Clofibrat, die bei der besonders stark wirkenden Verbindung gemäß Beispiel 22 $10^{+5}$ mal stärker als Clofibrat ist. Die Verbindung gemäß Beispiel 22 zeichnet sich überdies durch einen weiteren Vorzug aus, der in der Relation ihrer minimalen hypolipidämischen Wirkdosis in Bezug zur minimalen uterotrophen Wirkung liegt: er beträgt ≥ 30.

Bei den anderen aufgeführten Verbindungen liegt die minimale hypolipidämische Wirkdosis in etwa der

gleichen Größenordnung wie die uterotrophe Wirkung.

Tabelle I

| Veränderungen der Lipoproteine im Ratten-Serum in % nach 7-tägiger oraler Applikation der Verbindungen | | | | | | | |
|---|---|---|---|---|---|---|---|
| gemäß Beispiel Nr. | Dosierung mg/kg/Tag | Cholesterolveränderung in %, (bezogen auf die Kontrollgruppen)in den Serum-Lipoprotein-Fraktionen | | | | minimale Wirkdosis mg/kg/Tag | uterotrophe Wirkung mg/kg/Tag |
| | | VLDL | LDL | HDL | $\frac{HDL-C}{LDL-C}$ | | |
| 22 | 0,1 | -47 | -97 | -92 | | | |
| | 0,01 | -20 | -34 | - 8 | 1,38 | 0,01 | > 0,3 |
| 21 | 0,1 | -34 | -43 | -22 | 1,38 | | |
| | 0,03 | + 5 | -83 | -47 | 3,11 | 0,03 | > 0,01 |
| 19 | 0,3 | + 3 | -81 | -28 | 3,73 | | |
| | 0,1 | -18 | -84 | -85 | 0,94 | 0,1 | 0,1 |
| 9 | 30 | | -90 | -93 | | | |
| | 3 | | -85 | -84 | | 0,3 | |
| | 0,3 | -10 | -66 | -27 | 2,13 | | |
| 14 | 3 | -88 | -98 | -97 | | | |
| | 0,3 | -59 | -95 | -96 | 0,77 | 0,3 | 0,3 |
| 20 | 3 | -26 | -77 | -91 | | 0,3 | 0,3 |
| | 0,3 | -15 | -66 | -17 | 2,43 | | |
| 1 | 1 | - 6 | -49 | -22 | | 0,3 | 0,3 |
| | 0,3 | +12 | -34 | -17 | 1,27 | | |
| 18 | 3 | -38 | -93 | -95 | | 0,3 | 0,3 |
| | 0,3 | + 5 | -21 | -21 | 1,01 | | |
| 13 | 3 | -30 | -63 | -35 | | 0,3 - 1 | |
| | 0,3 | -12 | -12 | - 7 | 1,05 | | |
| 11 | 3 | -26 | -36 | -22 | 1,22 | 1 - 3 | |
| 5 | 3 | -52 | -53 | - 5 | 2,0 | 3 | |
| 15 | 3 | -50 | -93 | -96 | 0,65 | 3 | 3 |
| 17 | 3 | -41 | -92 | -59 | 5,05 | 3 | |
| 3 | 3 | -28 | -33 | + 4 | 1,55 | 3 | |
| 7 | 3 | - 8 | -20 | + 3 | 1,29 | 3 | |
| 12 | 3 | -51 | -33 | - 5 | 1,42 | 3 | |
| Clofibrat (Vergleichssubstanz) | 100 | -32 | -35 | -32 | 1,04 | 100 | keine |

Als therapeutische Zubereitung der Verbindungen der Formel I kommen vor allem Tabletten, Dragees, Kapseln, Suppositorien und Säfte in Frage. Die neuen Verbindungen können dabei entweder allein oder mit pharmakologisch annehmbaren Trägern vermischt, angewandt werden. Eine orale Anwendungsform wird bevorzugt. Zu diesem Zweck werden die aktiven Verbindungen vorzugsweise mit an sich bekannten Hilfsstoffen vermischt und durch an sich bekannte Methoden in geeignete Darreichungsformen gebracht, wie Tabletten, Steckkapseln, wäßrige oder ölige Suspensionen oder wäßrige oder ölige Lösungen. Als inerte Träger können z.B. Magnesiumkarbonat, Milchzucker oder Maisstärke unter Zusatz anderer Stoffe wie z.B. Magnesiumstearat verwendet werden. Dabei kann die Zubereitung als Trocken- oder als Feuchtgranulat erfolgen. Als ölige Trägerstoffe oder Lösungsmittel kommen besonders pflanzliche und tierische Öle in Betracht wie z.B. Sonnenblumenöl oder Lebertran. Als tägliche Dosis kommen etwa 1 mg bis 200 mg, vorzugsweise 2 mg bis 20 mg, bei Verwendung der Verbindung gemäß Beispiel 22 0,5 - 5 mg, bevorzugt 0,8 - 2 mg, in Betracht. Eine Dosierungseinheit enthält vorzugsweise 0,5 bis 2 mg Wirksubstanz gemäß Beispiel 22.

Die Zubereitungen können bei der Behandlung von Lipidstoffwechselstörungen außer den üblichen Füll- und Trägerstoffen noch ein Antihypertensivum, wie beispielsweise ein Saluretikum, Reserpin, Hydralazin,Guanethidin, α-Methyldopa, Clonidin, ACE-Hemmer oder ein β-Sympathikolytikum, eine antithrombotisch wirksame Verbindung, wie z.B. Acetylsalicylsäure, Sulfinpyrazon, Ticlopidin und Heparinoide, oder ein antihyperurikämisch wirksames Mittel, ein orales Antidiabetikum, ein Geriatrikum oder ein Mittel mit durchblutungssteigernder Wirkung enthalten.

Die folgenden Beispiele erläutern die Erfindung.

**Beispiel 1**

7-Diphenylmethylenbicyclo[2.2.1]heptan-2-oxim

33,7 g (129 mmol) 7-Diphenylmethylenbicyclo[2.2.1]heptan-2-on wurden mit 9,9 g (142 mmol) Hydroxylaminhydrochlorid und 17,8 g (129 mmol) Kaliumcarbonat in 50 ml Ethanol vier Stunden bei Raumtemperatur gerührt. Anschließend versetzte man mit 200 ml Wasser und extrahierte dreimal mit 100 ml Methylenchlorid. Die vereinigten Methylenchloridphasen wurden dreimal mit 300 ml Wasser gewaschen, über Natriumsulfat getrocknet und im Vakuum eingeengt. Man erhielt 31,7 g 7-Diphenylmethylenbicyclo-[2.2.1]heptan-2-oxim als amorphen Feststoff.

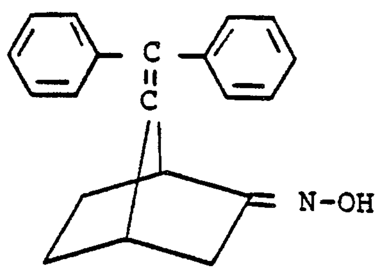

NMR ($CDCl_3$): 1,4-2,8 (m, 9H); 2,9-3,1 (m, 1H); 3,3-3,5 (m, 1H); 7,0-7,5 (m, 1OH); 7.85 (s, -OH)
Anal.: $C_{20}H_{19}NO$: 289,38
ber.:
C 83,01 H 6,62 N 4,84
gef.:
C 82,6 H 6,8 N 4,5

**Beispiel 2**

endo-7-(4,4′-Dichlordiphenylmethylen)bicyclo[2.2.1]heptan-2-carbonsäuredimethylamid

a) endo-7-(4,4′-Dichlordiphenylmethylen)bicyclo[2.2.1]hept-5-en-2-carbonsäure

35 g (117 mmol) 4,4′-Dichlordiphenylfulven und 16,8 g (234 mmol) Acrylsäure ließ man zwei Wochen bei Raumtemperatur miteinander reagieren. Die entstandene feste Masse wurde anschließend aus 220 ml Ethylacetat umkristallisiert. Man erhielt 21,8 g des endo-Addukts als weiße Kristalle vom Schmelzpunkt 182°C

b) endo-7-(4,4′-Dichlordiphenylmethylen)bicyclo[2.2.1]heptan-2-carbonsäure

21,7 g (58,5 mmol) der unter a) erhaltenen Carbonsäure wurden in 150 ml Tetrahydrofuran gelöst. Nach Zusatz von 1 g Pd/C wurde bei Raumtemperatur und Normaldruck bis zur Aufnahme von einem Equivalent Wasserstoff hydriert. Nach Absaugen des Katalysators und Abdestillieren des Lösungsmittels am Rotationsverdampfer blieben 22,1 g der hydrierten Säure als weiße Kristalle vom Schmelzpunkt 238°C zurück.

c) endo-7-(4,4′-Dichlordiphenylmethylen )bicyclo[2.2.1]-heptan-2-carbonsäuredimethylamid

22,0 g (59 mmol) der unter b) erhaltenen Carbonsäure wurden in 200 ml Toluol gelöst. Nach Zugabe von zwei Tropfen Pyridin als Katalysator tropfte man 11,3 g (95 mmol) Thionylchlorid zu und erhitzte das Gemisch drei Stunden lang zum Rückfluß. Danach wurden das Lösungsmittel sowie überschüssiges Thionylchlorid abdestilliert und der Rückstand in 200 ml Tetrahydrofuran gelöst. Zu der Lösung des Säurechlorids wurden anschließend bei 0°C 9,0 g (200 mmol) Dimethylamin hinzugefügt und über Nacht gerührt. Die Aufarbeitung des Reaktionsgemischs erfolgte analog Beispiel 1) mit Methylenchlorid/Wasser. Man erhielt 19,2 g endo-7-(4,4′-Dichlordiphenylmethylen)-bicyclo[2.2.1]heptan-2-carbonsäuredimethylamid als gelbliches Öl.

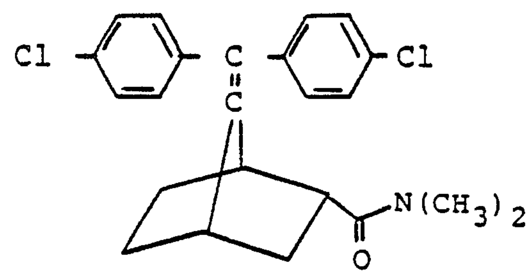

NMR ($CDCl_3$): 1,5-3,5 (m, 9H); 2,9 (s, $2CH_3$); 6,8-7,4 (dq,8H)

**Beispiel 3**

endo-2-Dimethylaminomethylen-7-(4,4′-dichlordiphenylmethylen)-bicyclo[2.2.1]heptan-Hydrochlorid

0,6 g (17,3 mmol) Lithiumaluminiumhydrid wurden in 20 ml Diethylether vorgelegt. Zu dieser Suspension tropfte man 9,2 g (23 mmol) endo-7-(4,4′-Dichlordiphenylmethylen)-bicyclo[2.2.1]heptan-2-carbonsäuredimethylamid in 50 ml Diethylether. Man erhitzte acht Stunden zum Rückfluß, ließ abkühlen und gab bei 0°C langsam verdünnte Salzsäure zu, bis ein pH-Wert von 7 erreicht wurde. Man versetzte mit 100 ml Wasser und trennte die Etherphase ab. Die Wasserphase wurde noch zweimal mit 50 ml Methylenchlorid extrahiert. Man vereinigte die Etherphase mit den Methylenchloridphasen, wusch zweimal mit 200 ml Wasser und trocknete über Natriumsulfat. Nach Abfiltrieren des Trockenmittels und Entfernen des Lösungsmittels blieben 8,8 g Reaktionsprodukt als Öl zurück.
Das Öl wurde in 100 ml Diethylether gelöst und da bei der Reduktion erhaltene Amin mit Chlorwasserstoff als Hydrochlorid ausgefällt. Man erhielt 5,0 g weiße Kristalle vom Schmelzpunkt 216°C.

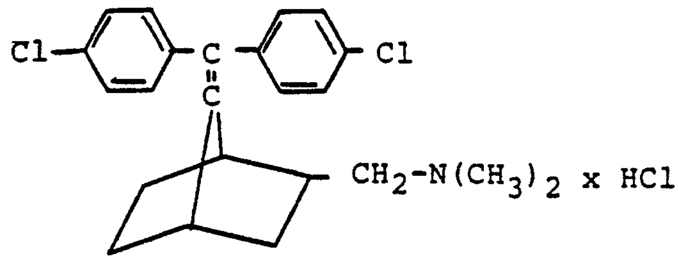

NMR (DMSO): 1,0-3,5 (m, 11H); 2,7 (s, $2CH_3$); 6,8-7,5 (dq,8H).
Anal.: $C_{24}H_{26}Cl_3N$: 434,85
ber.:
C 66,29 H 6,03 N 3,22 Cl 24,46
gef.:
C 65,1 H 5,9 N 3,1 Cl 24,2

In analoger Weise wurden die folgenden neuen Verbindungen hergestellt:

| Beispiel | A | B | $R^1$ | $R^2$ | $R^3$ | $R^4$ | Konfig. | Phys. Daten |
|---|---|---|---|---|---|---|---|---|
| 4 | - | 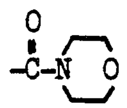 | H | H | Cl | H | endo | NMR: 1,4-3,2 (m, 11H), 3,2-4,0 (m,6H); 6,8-7,6 (m,9H) |
| 5 | - | 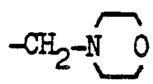 | H | H | Cl | H | endo | HCl-Salz: MP= 240°C |
| 6 | - | 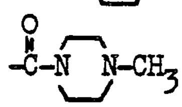 | H | H | Cl | Cl | endo | NMR: 1,5-4,0 (m, 17H), 2,25 (s,$CH_3$), 6,8-7,5 (dq,8H) |
| 7 | - | 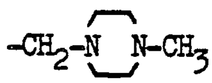 | H | H | Cl | Cl | endo | HCl-Salz: MP= 210°C |
| 8 | - | B + $R^1$ = NOH | | H | Cl | Cl | - | NMR: 1,5-3,5 (m, 8H), 6,8-7,5 (dq, 8H). |

nach beschriebenen Verfahren hergestellt

| Beispiel | A | B | $R^2$ | $R^1$ | $R^3$ | $R^4$ | Konfig. | Literatur |
|---|---|---|---|---|---|---|---|---|
| 9 | - | $-CN$ | H | H | H | H | endo | Ann. 566, 27 |
| 10 | = | $R^2$,B= 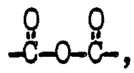, | | H | H | H | endo | " " |
| 11 | - | $R^2$,B= 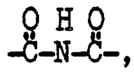, | | H | H | H | endo | J.Med.Pharm.Chem. 5, 883 (1962) |
| 12 | = | $-CN$ | H | H | H | H | exo | Synth. 1985, 798 |
| 13 | = | $-CN$ | H | H | H | H | endo | " " |
| 14 | = | $-CO_2H$ | H | H | H | H | exo | " " |
| 15 | - | $-CO_2H$ | H | H | H | H | endo | " " |
| 16 | = | $-CO_2CH_3$ | H | H | H | H | exo,endo | " " |

**Fortsetzung Tabelle:**

| Beispiel | A | B | $R^1$ | $R^2$ | $R^3$ | $R^4$ | Konfig. | Literatur |
|---|---|---|---|---|---|---|---|---|
| 17 | = | 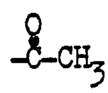 | H | H | H | H | endo | Synth. 1985, 798 |
| 18 | - | $-CN$ | Cl | H | H | H | exo(CN) | " " |
| 19 | - | $-OH$ | H | H | H | H | endo[1)] | EP-A- 0 158 144 |
| 20 | - | $-OH$ | H | H | H | H | exo[2)] | " " |
| 21 | - | $-CH_2OH$ | H | H | H | H | endo | " " |
| 22 | - | $B + R^1 = O$ | | H | H | H | - | " " |

1) MP = 120°C, 2) Öl

**Beispiel 23:** Tabletten

Tabletten, die für die orale Verabreichung geeignet sind, und die nachfolgend genannten Bestandteile enthalten werden auf an sich bekannte Weise hergestellt, indem man Wirk- und Hilfsstoffe granuliert und anschließend zu Tabletten verpreßt. Diese Tabletten eignen sich zur hypolipidämischen Behandlung in einer Dosis von ein bis zwei Tabletten 1-4 mal täglich.

| Bestandteile (pro Tablette) | Gewicht (mg) |
|---|---|
| Verbindung gemäß Beispiel 22 | 1 mg |
| Milchzucker | 50 mg |
| Maisstärke | 15 mg |
| Talkum | 1,5 mg |
| kolloidales Siliziumdioxid | 1,5 mg |
| Magnesiumstearat | 1 mg |

**Beispiel 24:** Kapseln

Kapseln, die für die orale Verabreichung geeignet sind, enthalten die nachfolgend genannten Bestandteile und können auf an sich bekannte Weise hergestellt werden, indem man Wirk- und Hilfsstoffe vermischt und in Gelatinekapseln abfüllt. Diese Kapseln dienen zur hypolipidämischen Behandlung in einer Dosis von ein bis zwei Kapseln 1 - 4 mal täglich.

| Bestandteile (pro Kapsel) | Gewicht (mg) |
|---|---|
| Verbindung gemäß Beispiel 22 | 0,5 mg |
| Milchzucker | 50 mg |
| Maisstärke | 15 mg |
| Talkum | 1,5 mg |
| kolloidales Siliziumdioxid | 1,5 mg |
| Magnesiumstearat | 1 mg |

**Ansprüche**

1. Pharmazeutisches Präparat enthaltend ein 7-Diphenylmethylenbicycloheptan bzw. -hepten der allgemeinen Formel I

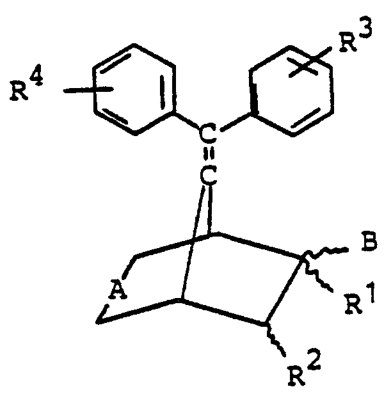

I

worin
A eine Einfach- oder Doppelbindung ist, B eine Carbonsäureamidgruppe der Formel

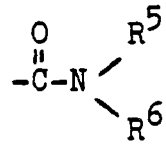

oder eine Methylenaminogruppe der Formel

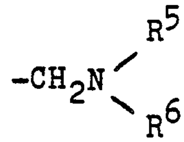

ist,

worin $R^5$ und $R^6$ gleich oder verschieden sind und Wasserstoff, oder $C_1$-$C_4$-Alkyl bedeuten oder worin $R^5$ und $R^6$ zusammen mit dem Stickstoffatom einen Morpholin-Piperazin- oder einen in 4-Stellung mit $C_1$-$C_4$-Alkyl substituierten Piperazinring bilden, oder B ist -OH, -CN, $-CO_2H$, $-CO_2$-($C_1$-$C_4$)-Alkyl oder $-\overset{\displaystyle O}{\overset{\|}{C}}-$($C_1$-$C_4$)-Alkyl,

$R^1$ Wasserstoff, Chlor, $C_1$-$C_4$-Alkyl oder zusammen mit B eine Oximgruppe (= N-OH) oder über eine Doppelbindung gebundener Sauerstoff (= O) ist,

$R^2$ Wasserstoff oder zusammen mit B ein Dicarbonsäureanhydrid $(-\overset{O}{\overset{\|}{C}}-O-\overset{O}{\overset{\|}{C}}-)$ oder ein Dicarbonsäureimid $(-\overset{O}{\overset{\|}{C}}-NH-\overset{O}{\overset{\|}{C}})$ ist,

$R^3$ und $R^4$ gleich oder verschieden sind und Wasserstoff, Halogen, ($C_1$-$C_4$)-Alkyl, ($C_1$-$C_4$)-Alkoxy, Trifluormethyl, Hydroxy, Amino, ($C_1$-$C_4$)-Alkylamino oder ($C_1$-$C_4$)-Dialkylamino bedeuten,

oder im Fall von basischen Substituenten für B deren physiologisch verträgliche Säureadditionssalze.

2. Verwendung einer Verbindung der Formel I oder eines physiologisch verträglichen Salzes als Arzneimittel.

3. Verwendung einer Verbindung der Formel I oder eines physiologisch verträglichen Salzes zur Herstellung von Arzneimitteln.

4. Hypolipidämikum enthaltend eine Verbindung der Formel I oder ein physiologisch verträgliches Säureadditionssalz.

5. Pharmazeutisches Präparat gemäß Anspruch 1, enthaltend als Wirkstoff eine Verbindung der Formel I, worin

A eine Einfachbindung

B eine $-CO_2H$, -CN, $-CH_2OH$ oder -OH-Gruppe

$R^1$ Wasserstoff oder zusammen mit B über eine Doppelbindung gebundener Sauerstoff (= O) oder die Oximgruppe (= N-OH)

$R^2$ Wasserstoff

$R^3$ und $R^4$ Wasserstoff oder Halogen

bedeuten.

6. 7-Diphenylmethylenbicycloheptane und 7-Diphenylmethylenbicycloheptene der Formel Ia

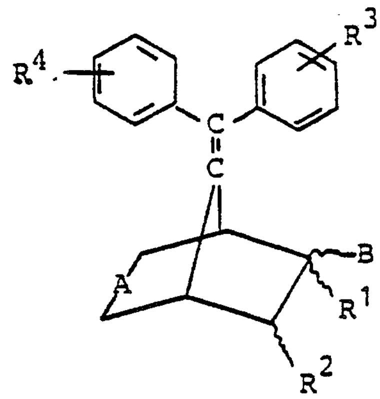

Ia

worin
A eine Einfach- oder Doppelbindung ist,
B eine Carbonsäureamidgruppe der Formel

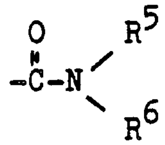

oder eine Methylenaminogruppe der Formel

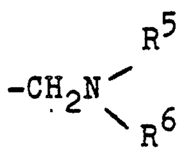

ist,
worin $R^5$ und $R^6$ gleich oder verschieden sind und Wasserstoff, oder $C_1$-$C_4$-Alkyl bedeuten, oder worin $R^5$ und $R^6$ zusammen mit dem Stickstoffatom einen Morpholin-Piperazin- oder einen in 4-Stellung mit $C_1$-$C_4$-Alkyl substituierten Piperazinring bilden,
$R^1$ Wasserstoff, Chlor oder zusammen mit B eine Oximgruppe (= N-OH) ist,
$R^2$ Wasserstoff darstellt und
$R^3$ und $R^4$ gleich oder verschieden sind und Wasserstoff, Halogen, ($C_1$-$C_4$)-Alkyl, ($C_1$-$C_4$)-Alkoxy, Trifluormethyl, Hydroxy, Amino, ($C_1$-$C_4$)-Alkylamino oder ($C_1$-$C_4$)-Dialkylamino bedeuten,
sowie deren physiologisch verträgliche Säureadditionssalze.

7. Verbindungen der Formel Ia, worin A eine Einfachbindung ist, B und $R^1$ gemeinsam die Oximgruppe bilden und $R^3$ und $R^4$ Wasserstoff oder Chlor bedeuten.

8. Verfahren zur Herstellung von Verbindungen der Formel Ia, dadurch gekennzeichnet, daß man
A) eine Bicycloheptan- oder Bicycloheptensäure der Formel Ib

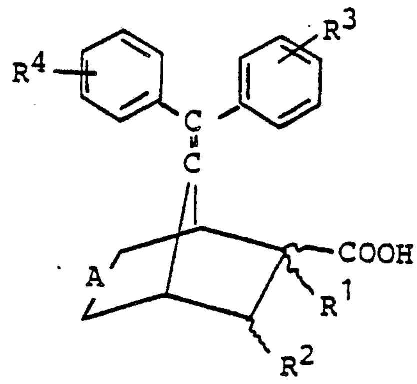

I b

worin A, $R^2$, $R^3$ und $R^4$ die zu Formel Ia angegebenen Bedeutungen haben und $R^1$ Wasserstoff oder Chlor ist mit einem Chlorierungsmittel in das entsprechende Säurechlorid überführt und dann mit einem Amin

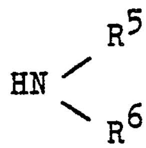

umsetzt zu einer Verbindung der Formel Ia worin A, $R^2$, $R^3$ und $R^4$ die angegebenen Bedeutungen haben, $R^1$ Wasserstoff oder Chlor ist und B die

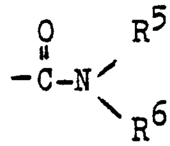

Gruppe, worin $R^5$ und $R^6$ die zu Formel Ia angegebenen Bedeutungen haben, darstellt und gegebenenfalls ein erhaltenes Carbonsäureamidderivat reduziert zu einer Verbindung der Formel Ia, worin A, $R^2$, $R^3$ und $R^4$ die zu Formel Ia angegebenen Bedeutungen haben, $R^1$ Wasserstoff oder Chlor ist und B die

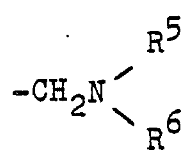

Gruppe, worin $R^5$ und $R^6$ die zu Formel Ia angegebenen Bedeutungen haben darstellt, oder

B) ein Keton der Formel Ic

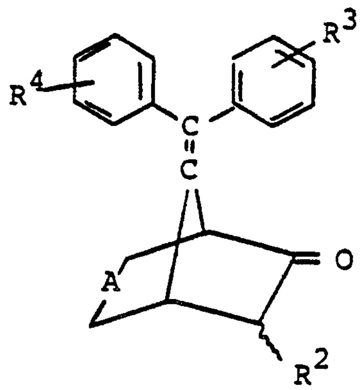

worin A, $R^2$, $R^3$ und $R^4$ die zu Formel Ia angegebenen Bedeutungen haben, mit Hydroxylaminhydrochlorid umsetzt zu einer Verbindung der Formel Ia, worin A, $R^2$, $R^3$ und $R^4$ die zu Formel Ia die angegebenen Bedeutungen haben und $R^1$ und B gemeinsam die =NOH-Gruppe bilden,
und gegebenenfalls in einer erhaltenen Verbindung der Formel Ia, worin A eine Doppelbindng darstellt, die Doppelbindung hydriert, und gegebenenfalls eine Verbindung der Formel Ia in ein physiologisch verträgliches Säureadditionssalz überführt.

Patentansprüche für die folgenden Vertragsstaaten: ES, GR

1. Verfahren zur Herstellung eines pharmazeutischen Präparates, dadurch gekennzeichnet, daß ein 7-Diphenylmethylenbicycloheptan bzw. -hepten der allgemeinen Formel I

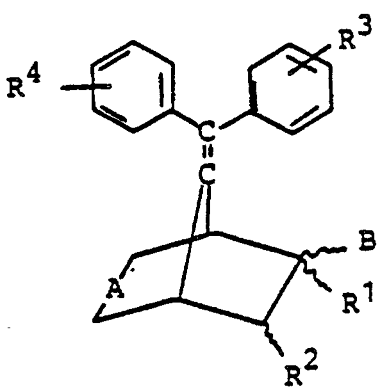

I

worin
A eine Einfach- oder Doppelbindung ist,
B eine Carbonsäureamidgruppe der Formel

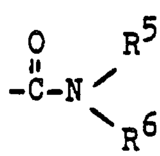

oder eine Methylenaminogruppe der Formel

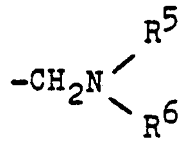

ist,
worin $R^5$ und $R^6$ gleich oder verschieden sind und Wasserstoff, oder $C_1$-$C_4$-Alkyl bedeuten oder worin $R^5$ und $R^6$ zusammen mit dem Stickstoffatom einen Morpholin-Piperazin- oder einen in 4-Stellung mit $C_1$-$C_4$-Alkyl substituierten Piperazinring bilden, oder B ist -OH, -CN, $-CO_2H$, $-CO_2-(C_1-C_4)$-Alkyl oder $-\underset{\overset{\|}{O}}{C}-(C_1-C_4)$-Alkyl,

$R^1$ Wasserstoff, Chlor, $C_1$-$C_4$-Alkyl oder zusammen mit B eine Oximgruppe (= N-OH) oder über eine Doppelbindung gebundener Sauerstoff (= O) ist,
$R^2$ Wasserstoff oder zusammen mit B ein Dicarbonsäureanhydrid
$(-\overset{O}{\overset{\|}{C}}-O-\overset{O}{\overset{\|}{C}}-)$ oder ein Dicarbonsäureimid
$(-\overset{O}{\overset{\|}{C}}-NH-\overset{O}{\overset{\|}{C}}-)$ ist,
$R^3$ und $R^4$ gleich oder verschieden sind und Wasserstoff, Halogen, $(C_1$-$C_4)$-Alkyl, $(C_1$-$C_4)$-Alkoxy, Trifluormethyl, Hydroxy, Amino, $(C_1$-$C_4)$-Alkylamino oder $(C_1$-$C_4)$-Dialkylamino bedeuten,
oder im Fall von basischen Substituenten für B deren physiologisch verträgliche Säureadditionssalze in eine geeignete Darreichungsform überführt wird.

2. Verfahren zur Herstellung eines Hypolipidämikums, dadurch gekennzeichnet, daß eine Verbindung der Formel I oder ein physiologisch verträgliches Säureadditionssalz in eine geeignete Darreichungsform überführt wird.

3. Verfahren zur Herstellung eines pharmazeutischen Präparates, dadurch gekennzeichnet, daß eine Verbindung der Formel I, worin
A eine Einfachbindung
B eine $-CO_2H$, -CN, $-CH_2OH$ oder -OH-Gruppe
$R^1$ Wasserstoff oder zusammen mit B über eine Doppelbindung gebundener Sauerstoff (= O) oder die Oximgruppe (= N-OH)
$R^2$ Wasserstoff
$R^3$ und $R^4$ Wasserstoff oder Halogen
bedeuten
in eine geeignete Darreichungsform überführt wird.

4. Verfahren zur Herstellung von 7-Diphenylmethylenbicycloheptanen und 7-Diphenylmethylbicycloheptenen der Formel Ia

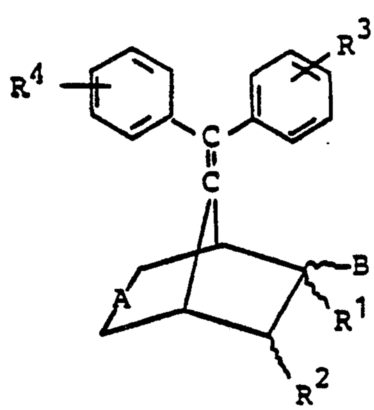

Ia

worin
A eine Einfach- oder Doppelbindung ist,
B eine Carbonsäureamidgruppe der Formel

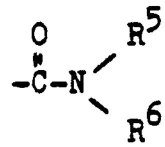

oder eine Methylenaminogruppe der Formel

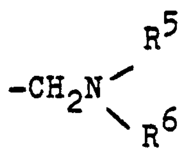

ist,
worin $R^5$ und $R^6$ gleich oder verschieden sind und Wasserstoff, oder $C_1$-$C_4$-Alkyl bedeuten, oder worin $R^5$ und $R^6$ zusammen mit dem Stickstoffatom einen Morpholin-Piperazin- oder einen in 4-Stellung mit $C_1$-$C_4$-Alkyl substituierten Piperazinring bilden.
$R^1$ Wasserstoff, Chlor oder zusammen mit B eine Oximgruppe (= N-OH) ist,
$R^2$ Wasserstoff darstellt und
$R^3$ und $R^4$ gleich oder verschieden sind und Wasserstoff, Halogen, ($C_1$-$C_4$)-Alkyl, ($C_1$-$C_4$)-Alkoxy, Trifluormethyl, Hydroxy, Amino, ($C_1$-$C_4$)-Alkylamino oder ($C_1$-$C_4$)-Dialkylamino bedeuten,
dadurch gekennzeichnet, daß man
A) eine Bicycloheptan- oder Bicycloheptensäure der Formel Ib

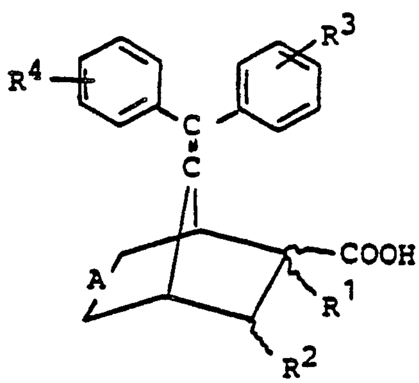

Ib

worin A, $R^2$, $R^3$ und $R^4$ die zu Formel Ia angegebenen Bedeutungen haben und $R^1$ Wasserstoff oder Chlor ist mit einem Chlorierungsmittel in das entsprechende Säurechlorid überführt und dann mit einem Amin

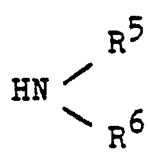

umsetzt zu einer Verbindung der Formel Ia worin A, $R^2$, $R^3$ und $R^4$ die angegebenen Bedeutungen haben, $R^1$ Wasserstoff oder Chlor ist und B die

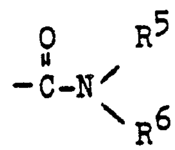

Gruppe, worin $R^5$ und $R^6$ die zu Formel Ia angegebenen Bedeutungen haben, darstellt und gegebenenfalls ein erhaltenes Carbonsäureamidderivat reduziert zu einer Verbindung der Formel Ia, worin A, $R^2$, $R^3$ und $R^4$ die zu Formel Ia angegebenen Bedeutungen haben, $R^1$ Wasserstoff oder Chlor ist und B die

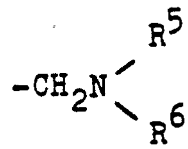

Gruppe, worin $R^5$ und $R^6$ die zu Formel Ia angegebenen Bedeutungen haben darstellt, oder
B) ein Keton der Formel Ic .

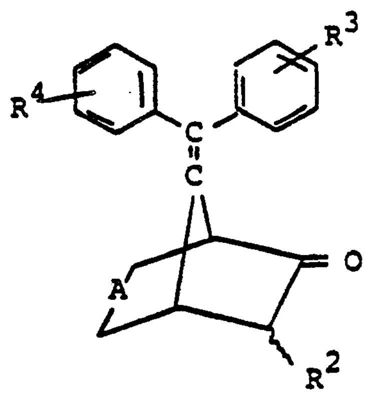

worin A, $R^2$, $R^3$ und $R^4$ die zu Formel Ia angegebenen Bedeutungen haben, mit Hydroxylaminhydrochlorid umsetzt zu einer Verbindung der Formel Ia, worin A, $R^2$, $R^3$ und $R^4$ die zu Formel Ia die angegebenen Bedeutungen haben und $R^1$ und B gemeinsam die =NOH-Gruppe bilden,
und gegebenenfalls in einer erhaltenen Verbindung der Formel Ia, worin A eine Doppelbindung darstellt, die Doppelbindung hydriert, und gegebenenfalls eine Verbindung der Formel Ia in ein physiologisch verträgliches Säureadditionssalz überführt.

5. Verfahren gemäß Anspruch 4, dadurch gekennzeichnet, daß man eine Verbindung der Formel Ia herstellt, in der A eine Einfachbindung ist, B und $R^1$ gemeinsam die Oximgruppe bilden und $R^3$ und $R^4$ Wasserstoff oder Chlor bedeuten.